# DEMANDE DE BREVET EUROPEEN

(11) **EP 1 656 970 A1**
(43) Date de publication de la demande: **17.05.2006**
(21) Numéro de dépôt: 05292297.8
(22) Date de dépôt: 28.10.2005
(51) Int. Cl.: A61Q 19/08, A61K 8/64, A61K 8/97, A61K 8/60, A61K 8/99, A61K 8/98

(54) **Composition cosmétique comprenant un hydrolysat de protéines de riz et un agent augmentant la synthèse des glycosaminoglycanes**

(30) Priorité: 10.11.2004 FR 0452593
(71) Demandeur: L'OREAL, 75008 Paris (FR)
(72) Inventeur: Guillou, Véronique, 92160 Antony (FR); Thiry, Annabelle, 75013 Paris (FR)
(74) Mandataire: Leonard, Armelle

(57) **Abrégé**

La présente invention se rapporte à un procédé cosmétique pour prévenir et/ou diminuer les signes du vieillissement cutané, en particulier remodeler le visage et/ou limiter le creusement du visage dû à l'âge, par application sur la peau du visage d'une composition renfermant, dans un milieu physiologiquement acceptable, au moins un hydrolysat de protéines de riz et au moins un agent augmentant la synthèse des glycosaminoglycanes.

Elle concerne également une composition cosmétique renfermant, dans un milieu physiologiquement acceptable, au moins un hydrolysat de riz et au moins un agent augmentant la synthèse des glycosaminoglycanes choisi parmi un extrait de *Laminaria ochroleuca* et le C-β-D-xylopyranoside-2-hydroxy-propane, ses sels et isomères optiques et géométriques.

## Description

La présente invention se rapporte à un procédé cosmétique pour prévenir et/ou diminuer les signes du vieillissement cutané, en particulier remodeler le visage et/ou limiter le creusement du visage dû à l'âge, par application sur la peau du visage d'une composition renfermant, dans un milieu physiologiquement acceptable, au moins un hydrolysat de protéines de riz et au moins un agent augmentant la synthèse des glycosaminoglycannes.

Elle porte également sur une composition cosmétique renfermant dans un milieu physiologiquement acceptable au moins un hydrolysat de protéines de riz et au moins un agent augmentant la synthèse des glysaminoglycannes choisi parmi un extrait de *Laminaria ochroleuca* et le C-β-D-xylopyranoside-2-hydroxy-propane, ses sels et isomères optiques et géométriques.

Elle concerne également l'utilisation cosmétique d'au moins un hydrolysat de protéines de riz associé à au moins un agent augmentant la synthèse des glycosaminoglycannes dans une composition, comme agent destiné à remodeler le visage et/ou limiter le creusement du visage dû à l'âge.

Il est connu que les signes du vieillissement cutané ont à la fois des causes chronologiques (vieillissement génétiquement programmé), et des facteurs additionnels aggravants tels que l'exposition au rayonnement UV et surtout les carences hormonales survenant lors de la ménopause. Ils se traduisent par des modifications de l'état de la peau qui sont essentiellement dues à un ralentissement et à un déséquilibre du fonctionnement cutané, qui se traduit par une « atrophie » de l'ensemble des assises de la peau. On observe ainsi une diminution de la qualité du derme (élastine, collagène, glycosaminoglycannes) et une perte de consistance de la matrice extracellulaire ; une diminution de l'épaisseur de l'épiderme (ralentissement du renouvellement cellulaire) ; un ralentissement de la production de lipides et des protéines de structure épidermique ; un déséquilibre de la desquamation ; et une réduction du contenu en eau de la peau.

En particulier, par suite des modifications précitées, le matelas de soutien de la peau s'affaiblit et la masse adipeuse fond : ainsi, le visage se creuse progressivement et la peau à tendance à tomber. Ce phénomène est surtout visible au niveau des joues, qui se creusent de plus en plus, du contour de l'oeil, et de l'ovale du visage, qui est moins bien dessiné (apparition de bajoues).

Or, on cherche constamment des moyens de contrer, ou tout du moins de retarder, l'apparition de ces signes de vieillissement et de paraître ainsi plus jeune plus longtemps.

Pour limiter cet affaissement de la peau et l'apparition des creux mentionnés précédemment, un moyen bien connu consiste à renforcer l'architecture du derme au moyen d'actifs stimulant la synthèse de collagène et de glycosaminoglycannes (ci-après, GAG). Des actifs classiquement utilisés à cette fin sont notamment : l'acide ascorbique et ses dérivés, les extraits de *Centella asiatica,* les rétinoïdes, certains hydrolysats de protéines de soja et les auxines (qui augmentent la synthèse de collagène), ainsi que les extraits d'algue brune *Padina pavonica* et de *Saccharomyces cerevisiae* (qui augmentent la synthèse des GAG). Cependant, le résultat obtenu par cette voie n'est pas totalement satisfaisant.

Or, la Demanderesse a découvert, de manière surprenante et inattendue, que des hydrolysats de protéines de riz comprenant des peptides de faible poids moléculaire (inférieur à 1400 daltons) comme ceux décrits dans WO02/102347 et/ou des peptides de poids moléculaire supérieur à 1400 daltons, avaient la propriété d'inhiber la lipolyse et ainsi d'éviter ou de ralentir la fonte des graisses contenues dans les tissus de soutien de la peau.
C'est dans ce contexte que la Demanderesse a imaginé utiliser un hydrolysat de protéines de riz en association avec un agent augmentant la synthèse des glycosaminoglycannes en vue de lutter efficacement contre les signes de vieillissement cutané et en particulier contre les divers mécanismes de creusement et d'affaissement du visage.

On connaît de l'art antérieur l'utilisation d'hydrolysats de protéines de riz dans des compositions cosmétiques pour le cheveu ou la peau, notamment comme agent hydratant (EP 1 090 630) ou comme agent anti-rides (WO02/102347).
Mais à la connaissance de la Demanderesse, il n'a jamais été suggéré l'utilisation d'un hydrolysat de riz en association avec un agent augmentant la synthèse des glysosaminoglycannes pour lutter contre les signes du vieillissement cutané, et en particulier pour remodeler le visage et/ou limiter le creusement du visage dû à l'âge.

La présente invention a donc pour objet un procédé cosmétique pour prévenir et/ou diminuer les signes du vieillissement cutané, par application sur la peau du visage d'une composition renfermant, dans un milieu physiologiquement acceptable, au moins un hydrolysat de protéines de riz et au moins un agent augmentant la synthèse des glycosaminoglycannes.

En particulier, le procédé cosmétique selon l'invention vise à remodeler le visage et/ou limiter le creusement du visage dû à l'âge.

Plus spécifiquement, le procédé cosmétique selon l'invention vise à redessiner les contours du visage et/ou limiter le creusement des joues et/ou du contour de l'oeil.

L'hydrolysat de protéines de riz utilisé dans le procédé de l'invention pourra comprendre des peptides de faible poids moléculaire (inférieur à 1400 daltons) et/ou des peptides de poids moléculaire supérieur à 1400 datons.
En particulier, on utilisera un hydrolysat de protéines de riz comprenant des peptides ayant une masse moléculaire inférieure ou égale à 5000 daltons, ou préférentiellement une masse moléculaire inférieure ou égale à 3000 Daltons.

Un procédé de préparation de cet hydrolysat de protéines de riz comprend par exemple les étapes consistant à :
- broyer des grains de riz (ex : *Oryza sativa*),
- solubiliser des protéines de riz dans l'eau pour obtenir une suspension,
- hydrolyser cette suspension en présence d'une ou plusieurs protéases;
- avantageusement filtrer sur membrane pour éliminer les composés insolubles, l'enzyme résiduelle et les peptides de haut poids moléculaire (supérieur à 10.000 daltons) ;
- et éventuellement réaliser une filtration stérilisante de ce concentrat.

Selon un mode particulier de l'invention, l'hydrolysat de protéines de riz utilisé dans le procédé de l'invention contient des peptides de masse moléculaire moyenne allant de 1000 daltons à 5000 daltons, de préférence une masse moléculaire moyenne allant de 2000 daltons à 3000 daltons.

Un procédé de préparation de cet hydrolysat de protéines de riz comprend par exemple les étapes consistant à :
- broyer des grains de riz (ex : *Oryza sativa*),
- solubiliser des protéines de riz dans l'eau pour obtenir une suspension,
- hydrolyser cette suspension en présence d'une ou plusieurs protéases;
- filtrer sur membrane pour éliminer les composés insolubles, l'enzyme résiduelle et les peptides de haut poids moléculaire, afin de concentrer la fraction active en peptides de masse moléculaire allant de 1000 à 5000 daltons, de préférence de 2000 à 3000 daltons ;
- et éventuellement réaliser une filtration stérilisante de ce concentrat.

Avantageusement, le procédé de préparation de cet hydrolysat de protéines de riz ne comprend pas d'étape de fermentation ou de germination. En outre, le riz utilisé dans la composition selon l'invention et/ou mis en oeuvre dans le procédé ci-dessus appartient de préférence au genre *Oryza* et est plus préférentiellement de l'espèce *Oryza sativa L.* Avantageusement, l'hydrolysat de riz utilisé dans la composition selon l'invention, et celui obtenu dans le procédé de préparation ci-dessus, sont issus d'une fraction de riz exempte de son de riz.

Cet hydrolysat de protéines de riz pourra notamment être défini par le profil en acides aminés suivant (% par rapport au % total d'acides aminés) :

| | | | |
|---|---|---|---|
| Ile | 4.5 | Met | 2.3 |
| Leu | 8.4 | Lys | 4.5 |
| Val | 6.3 | Arg | 9.0 |
| Met | 2.3 | Phe | 5.8 |
| Ala | 5.6 | Tyr | 5.3 |
| Gly | 4.3 | Glu | 17.5 |
| Ser | 5.5 | Asp | 10.0 |
| Thr | 3.9 | Cys | 0.9 |
| Pro | 3.9 | | |

Un tel hydrolysat de protéines de riz est référencé dans le Chemical Abstract sous le CAS n° 94350-05-7 ; il est notamment disponible dans le commerce auprès de la société PENTAPHARM sous la dénomination commerciale Colhibin®.

Selon un autre mode de réalisation de l'invention, l'hydrolysat de protéines de riz utilisé dans le procédé de l'invention est enrichi en peptides qui ont une masse moléculaire inférieure à 1400 Daltons.
De préférence, plus de 50% et, mieux, plus de 70%, des peptides contenus dans l'hydrolysat de protéines de riz ont une masse moléculaire inférieure à 1400 Daltons. En outre, les peptides contenus dans l'hydrolysat de protéines de riz ont de préférence une masse moléculaire moyenne comprise entre 300 et 400 Daltons, avantageusement entre 350 et 370 Daltons. Enfin, on préfère qu'au moins 40% des peptides ayant une masse moléculaire inférieure à 1400 Daltons aient une masse moléculaire comprise entre 75 et 175 Daltons ('peptides courts'). En général, moins de 60%, voire moins de 50%, des peptides ayant une masse moléculaire inférieure à 1400 Daltons auront une masse moléculaire comprise entre 75 et 175 Daltons.

En d'autres termes, on préfère qu'au moins 20%, et plus préférentiellement au moins 30%, des peptides contenus dans cet hydrolysat de protéines de riz aient une masse moléculaire comprise entre 75 et 175 Daltons. Ces peptides de faible masse moléculaire représenteront généralement moins de 50%, voire moins de 40%, des peptides contenus dans l'hydrolysat de protéines de riz. Ils sont de préférence essentiellement constitués de mono-, di- et tripeptides.

Un tel hydrolysat est notamment disponible dans le commerce auprès de la société SILAB sous la dénomination commerciale Nutriskin. Il peut être obtenu comme décrit dans la demande WO 02/102347.

Un procédé de préparation de cet hydrolysat de protéines de riz comprend par exemple les étapes consistant à :
- solubiliser des protéines de riz dans l'eau pour obtenir une suspension,
- hydrolyser cette suspension en présence d'une ou plusieurs enzymes choisies parmi : les aminopeptidases bactériennes en milieu neutre ou acide, les protéases stomacales d'origine animale en milieu acide, les protéases bactériennes en milieu neutre, les protéases bactériennes en milieu alcalin, extraites de *Bacillus licheniformis* ou de *Bacillus subtilis*, les endoprotéases bactériennes, et les enzymes pancréatiques d'origine animale ;
- concentrer la fraction active en peptides de poids moléculaire inférieur à 1400 Daltons ;
- et éventuellement réaliser une filtration stérilisante de ce concentrat.

Avantageusement, ce procédé ne comprend pas d'étape de fermentation ou de germination. En outre, le riz utilisé dans la composition selon l'invention et/ou mis en oeuvre dans le procédé ci-dessus appartient de préférence au genre *Oryza* et est plus préférentiellement de l'espèce *Oryza sativa L.* Avantageusement, l'hydrolysat de riz utilisé dans la composition selon l'invention, et celui obtenu dans le procédé ci-dessus, sont issus d'une fraction de riz exempte de son de riz.

Les extraits peptidiques de riz sont généralement mis en oeuvre dans la présente invention en une quantité efficace pour inhiber *in vitro* l'hydrolyse des triglycérides adipocytaires, selon un test analogue à celui présenté à l'Exemple 1 ci-après.

Il représente ainsi par exemple de 0,01 à 2% en poids (en matière active) et, mieux, de 0,02 à 0,4% en poids (en matière active), par rapport au poids total de la composition.
De préférence, il représente de 0,01 à 0,5% en poids (en matière active) et, mieux, de 0,02 à 0,2% en poids (en matière active), par rapport au poids total de la composition.

Un autre constituant essentiel de la composition utilisé dans le procédé de l'invention est un agent augmentant la synthèse des glycosaminoglycannes.

Comme agents augmentant la synthèse des glycosaminoglycannes, on peut notamment utiliser selon l'invention : le D-xylose, ses esters et les oligosaccharides contenant du D-xylose tels que décrits notamment dans la demande WO99/24009 ; les C-glycosides décrits dans la demande WO 02-051828, tels que le C-β-D-xylopyranoside-2-hydroxy-propane et ses sels et isomères optiques et géométriques ; un produit de fermentation du lait par *Lactobacillus vulgaris*, tel que celui commercialisé par la société BROOKS sous la dénomination commerciale Biomin yogourth® ; un extrait d'algue brune *Padina pavonica* tel que celui commercialisé par la société ALBAN MÜLLER sous la dénomination commerciale HSP3® ; un extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que celui disponible auprès de la société SECMA sous la dénomination commerciale Laminaïne® ; un extrait de *Centella asiatica* tel que celui disponible auprès de la société ROCHE sous la dénomination commerciale ETCA® ; et un extrait de cresson *(Nasturtium officinale*) disponible auprès de la société SILAB sous la dénomination commerciale Odraline®.

De préférence, on utilisera au moins un agent augmentant la synthèse des glycosaminoglycannes choisi parmi le D-xylose, ses esters et des oligosaccharides contenant du D-xylose, les C-glycosides décrits dans la demande WO 02-051828 tels que le C-β-D-xylopyranoside-2-hydroxy-propane et ses sels et isomères optiques et géométriques, un extrait d'algue brune *Padina pavonica,* un extrait de *Laminaria ochroleuca* et un extrait de cresson (*Nasturtium officinale*).

Dans le cadre de cette invention, on préfère utiliser comme agent augmentant la synthèse des glycosaminoglycanes, un extrait de *Laminaria ochroleuca* ou le C-β-D-xylopyranoside-2-hydroxy-propane et ses sels et isomères optiques et géométriques.

*Laminaria ochroleuca* est une variété de laminaire, de couleur jaune-marron, qui est connue pour lutter contre le stress oxydant et le vieillissement cellulaire et en particulier pour favoriser la synthèse de glycosaminoglycannes (EP-1 074 262).

L'extrait de *Laminaria ochroleuca* est notamment susceptible d'être obtenu suivant un procédé tel que décrit dans la demande EP-1 074 262, et qui comprend par exemple les étapes consistant à :
- récolter des algues fraîches, par exemple sur les côtes nord de la Bretagne,
- les laver,
- les broyer, avantageusement jusqu'à une taille comprise entre 100 et 200 µm,
- éliminer les débris cellulaires par centrifugation, pour obtenir un premier surnageant,
- fractionner le surnageant par précipitation à l'acide, de préférence à l'acide chlorhydrique, et par adsorption sur charbon activé, pour obtenir un second surnageant,
- filtrer le second surnageant (par exemple au moyen d'une filtration tangentielle avec un seuil de coupure de 1000 Daltons) jusqu'à ce que le filtrat ait atteint une concentration en glycine-bétaïne, mesurée par HPLC, de 12.5% à 2.5%.

Les C-glycosides tels que le C-β-D-xylopyranoside-2-hydroxy-propane, ses sels et isomères optiques et géométriques sont décrits dans la demande WO 02-051828, incorporée par référence dans la présente demande.

L'agent augmentant la synthèse des glycosaminoglycannes peut représenter de 0,001 à 5% en poids (en matière active) et de préférence de 0,01 à 1% en poids (en matière active), par rapport au poids total de la composition. Dans le cas où il s'agit d'un extrait de *Laminaria ochroleuca*, cet agent représente avantageusement de 0,005 à 0,1 % (en poids d'extrait sec) et de préférence de 0,01 à 0,05% (en poids d'extrait sec) du poids total de la composition.

Dans ce qui précède, on entend par "matière active" un composé ou un mélange de composés dépourvus du solvant dans lequel, et des additifs (tels que des conservateurs) avec lesquels, ils sont commercialisés et du solvant dans lequel ils ont été synthétisés ou au moyen duquel ils ont été extraits.

L'invention porte également sur une composition cosmétique susceptible d'être utilisée dans le procédé selon l'invention renfermant, dans un milieu physiologiquement acceptable, au moins un hydrolysat de protéines de riz et au moins un extrait de *Laminaria ochroleuca*.

Elle porte également sur une composition cosmétique susceptible d'être utilisée dans le procédé selon l'invention renfermant, dans un milieu physiologiquement acceptable, au moins un hydrolysat de protéines de riz et au moins le C-β-D-xylopyranoside-2-hydroxy-propane et ses sels et isomères optiques et géométriques.

L'hydrolysat de protéines de riz ainsi que l'extrait de *Laminaria ochroleuca* et les C-glycosides utilisables dans ladite composition sont définis précédemment dans la description.

L'hydrolysat de protéines de riz représente dans ces compositions par exemple de 0,01 à 2% en poids (en matière active) et, mieux, de 0,02 à 0,4% en poids (en matière active), par rapport au poids total de la composition.
De préférence, il représente de 0,01 à 0,5% en poids (en matière active) et, mieux, de 0,02 à 0,2% en poids (en matière active), par rapport au poids total de la composition.
L'extrait de *Laminaria ochroleuca* de 0,001 à 5% en poids (en matière active) et de préférence de 0,01 à 1 % en poids (en matière active), par rapport au poids total de la composition. Dans le cas où il s'agit d'un extrait de *Laminaria ochroleuca*, cet agent représente avantageusement de 0,005 à 0,1 % (en poids d'extrait sec) et de préférence de 0,01 à 0,05% (en poids d'extrait sec) du poids total de la composition.

Les C-glycosides et en particulier le C-β-D-xytopyranoside-2-hydroxy-propane, ses sels et isomères optiques et géométriques représente par exemple de 0,001 à 5% en poids (en matière active) par rapport au poids total de la composition, et de préférence de 0,01 à 1% en poids (en matière active).

La composition selon l'invention est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et ne génère pas d'inconforts inacceptables (picotements, tiraillements, rougeurs), susceptible de détourner la consommatrice d'utiliser cette composition.

La composition selon l'invention peut se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion huile-dans-eau (H/E). En outre, selon une variante de l'invention, l'hydrolysat de protéines de riz et l'agent augmentant la synthèse des glycosaminoglycannes sont incorporés ensemble, ou chacun séparément, dans un vésicule du type liposome ou du type niosome, en particulier dans des niosomes tels que décrits notamment dans la demande EP-0 582 503. Ces niosomes sont formés à partir d'un ester ou éther de polyol, tel qu'un ester d'acide gras et de sucrose, en particulier le distéarate de sucrose ; d'un lipide amphiphile anionique ou cationique, en particulier anionique, tel qu'un sel (notamment disodique) de stéaroyl glutamate ; et d'un stérol tel que le cholestérol.

Comme huiles utilisables dans la composition selon l'invention, on peut citer : les esters d'acides gras et d'alcools gras ; les huiles de silicone, volatiles (telles que les cyclomethicones) ou non volatiles (telles que les dimethicones) ; les alcools gras ramifiés tels que l'octyldodécanol ; les huiles hydrocarbonées telles que la vaseline, le squalane, l'isohexadécane et les huile minérales ; les huiles végétales ; et le beurre de karité. La phase huileuse peut également comprendre des cires telles que la cire d'abeille.

Cette composition peut en outre contenir divers adjuvants couramment utilisés dans le domaine cosmétique, tels que des polyols, en particulier la glycérine, le propylène glycol et les polyéthylène glycols ; des émulsionnants dont les esters d'acides gras et de polyéthylène glycol, les esters d'acides gras et de glycéryle, les esters d'acides gras et de sucrose, les éthers d'alcools gras et de polyéthylène glycol, et les esters d'acides gras et de méthylglucose éventuellement oxyéthylénés ; des co-émulsionnants tels que les alcools cétylique et stéarylique ; des charges, notamment de la silice, et des poudres expansées telles que les microsphères formées d'un terpolymère de chlorure de vinylidène, d'acrylonitrile et de méthacrylate et commercialisées sous la dénomination EXPANCEL par la société Kemanord Plast ; des épaississants et/ou gélifiants tels que les gommes de polysaccharides ou de silicone, les homo- et copolymères d'acrylamide, les homo- et copolymères d'acide acrylique et les homo- et copolymères d'acide acrylamido méthylpropane sulfonique (AMPS) ; des conservateurs ; des séquestrants ; des ajusteurs de pH tels que la triéthanolamine, l'hydroxyde de sodium ou l'acide citrique ; de l'éthanol ; des colorants ; des nacres ; et des parfums.

Bien entendu, l'homme du métier veillera à choisir ce ou ces éventuels composés additionnels et/ou leur quantité de manière telle que les propriétés avantageuses de la composition selon l'invention ne soient pas, ou substantiellement pas, altérées par l'adjonction envisagée.

La composition selon l'invention permet de ralentir la perte de matière du visage responsable de son creusement et de son affaissement avec l'âge. Pour renforcer les effets de cette composition, celle-ci peut renfermer au moins un composé choisi parmi :
- les agents tenseurs tels que tels que :
   (1) les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
   (2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
   (3) les protéines et hydrolysats de protéines végétales, en particulier de maïs, de seigle, de froment, de sarrasin, de sésame, d'épautre, de pois, de fève, de lentille, de soja et de lupin,
   (3) les silicates mixtes, notamment les phyllosilicates et en particulier les Laponites,
   (4) les microparticules de cire, choisies par exemple parmi les cires de Carnauba, de Candelila ou d'Alfa,
   (5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane ;
- les agents dermo-relaxants tels que l'adénosine, les sels de magnésium et de manganèse (en particulier les gluconates) et les extraits de Dioscorea opposita ou Dioscorea villosa contenant de la diosgénine ;
- les agents augmentant la synthèse de protéines de la jonction dermo-épidermique, telles que la laminine, le collagène IV et/ou le collagène VII, et en particulier l'acide ascorbique ou un hydrolysat de malt, obtenu par hydrolyse enzymatique, tel que celui commercialisé sous la dénomination commerciale Basaline par la société COLETICA ;
- les agents hydratants qui peuvent être soit des agents humectants tels que l'acide hyaluronique et ses sels, soit des agents stimulant la différenciation des kératinocytes et renforçant ainsi la barrière cutanée pour éviter le dessèchement de la peau, tels que : un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol ;
- des agents desquamants tels que les α- et les β-hydroxyacides, en particulier l'acide salicylique et ses dérivés tels que l'acide n-octanoyl-5-salicylique ; et un extrait de bourgeons de hêtre *Fagus sylvatica* tel que celui commercialisé par la société GATTEFOSSE sous la dénomination commerciale Gatuline® ; et
- les composés augmentant la synthèse de collagène et de pro-collagènes, tels que le mannuronate de monométhylsilanetriol, commercialisé notamment par la société EXSYMOL sous la dénomination commerciale Algisium C.

La composition selon l'invention peut en outre contenir au moins un filtre UVA et/ou UVB. Les filtres solaires peuvent être choisis parmi les filtres organiques, les filtres inorganiques et leurs mélanges.

Les filtres organiques plus particulièrement préférés sont choisis parmi les suivants (cités selon la nomenclature CTFA) : Ethylhexyl Salicylate, Homosalate, Ethylhexyl Methoxycinnamate, Butyl Methoxydibenzoylmethane, Octocrylene, Phenylbenzimidazole Sulfonic Acid, Disodium Phenyl Dibenzimidazole Tetra-sulfonate, Benzophenone-3, Benzophenone-4, Benzophenone-5, 4-Methylbenzylidene camphor, Terephthalylidene Dicamphor Sulfonic Acid, Bis-Ethylhexyloxyphenol Methoxyphenyl Triazine, Ethylhexyl triazone, Diethylhexyl Butamido Triazone, Methylène bis-Benzotriazolyl Tetramethylbutylphénol, Drometrizole Trisiloxane, Polysilicone-15, et parmi les suivants (cités en noms chimiques) : le 2-(4-diethylamino-2-hydroxybenzoyl)-benzoate de n-hexyle, le 1,1-dicarboxy (2,2'-diméthyl-propyl)-4,4-diphénylbutadiène, la 2,4-bis-[5-1 (diméthylpropyl)benzoxazol-2-yl-(4-phenyl)-imino]-6-(2-ethylhexyl)-imino-1,3,5-triazine, et leurs mélanges.

Les filtres inorganiques sont choisis parmi des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium.

L'invention concerne encore l'utilisation cosmétique d'au moins un hydrolysat de protéines de riz associé à au moins un agent augmentant la synthèse des glycosaminoglycannes dans une composition, comme agent destiné à remodeler le visage et/ou limiter le creusement du visage dû à l'âge.

L'invention sera maintenant illustrée par les exemples non limitatifs suivants. Dans ces exemples, les quantités sont indiquées en pourcentage pondéral.

### EXEMPLES

### Exemple 1 : Mise en évidence des propriétés d'un hydrolysat de protéines de riz sur la lipolyse

Nous avons évalué l'effet d'un hydrolysat de protéines de riz selon l'invention (Nutriskin de SILAB, défini dans la demande WO 02/102347, à 6.25% de matière active) sur l'hydrolyse des triglycérides adipocytaires en mesurant la libération d'acides gras non estérifiés (AGNE) dans le milieu.
Cet hydrolysat de protéines de riz comprend des peptides de faible masse moléculaire (inférieure à 1400 daltons) .
Deux références ont été utilisées, la Théophylline (qui stimule la production AMPcyclique) et l'Isoprotérénol (qui est un agoniste béta-adrénergique). Ces deux molécules ont un effet lipolytique c'est-à-dire qu'elles augmentent la libération d'acides gras non estérifiés par les adipocytes.

### Mode opératoire :

Les adipocytes ont été isolés à partir d'une plastie abdominale puis incubés en présence de collagènase ensuite lavés et repris avec du milieu d'essai. Ce milieu d'essai comportait :
- Produit à tester (hydrolysat représentant 0.0625 % ou 0.0125 % en matière active / Théophylline 1mM/ Isoprotérénol 1µM), ou rien pour le témoin
- bicarbonate 1.87 mg/ml
- pénicilline / streptomycine 25Ul/ml/25µg/ml
- glutamine 2mM
- MEM sans rouge phénol qsp100% (v/v) additionné d'albumine bovine sérique délipidée à 0.5 % (p/v)

Nous avons ensuite ajouté la suspension d'adipocytes et les mélanges ont été incubés à 37°C. Les AGNE ont été dosés dans les fractions de milieu sous-adipocytes, après décantation. Le dosage a été réalisé à l'aide d'un kit NEFA-C selon les directives du fournisseur.

Deux contrôles (un positif et un négatif) d'absence d'interférence du produit avec le dosage des AGNE ont été réalisés.

**Résultats :**

| **Traitement** | **Concentration** | **AGNE (µM)** | **% activité vs Témoin** | ***p*** |
|---|---|---|---|---|
| Témoin non traité | - | 78.8 | 100 | P<0.01 |
| Hydrolysat de protéines de riz | 0.0625 % | 32.0 | 41 | P<0.01 |
| | 0.0125 % | 16.8 | 21 | P<0.01 |
| Théophylline | 1 mM | 496.3 | 630 | P<0.01 |
| Isoprotérénol | 1µM | 472.3 | 600 | P<0.01 |

Dans cet essai, la lipolyse basale était élevée (79 µM AGNE libérés en 2 heures dans ces conditions). La Théophylline à 1 mM et l'Isoprotérénol à 1 µM ont stimulé de façon significative la lipolyse (respectivement 630 % et 600 % du témoin), ce qui a permis de valider l'essai. L'hydrolysat de protéines de riz a diminué significativement la lipolyse basale, respectivement à 21 % et 41 % du témoin, et cela sans interférence apparente du produit avec le dosage des AGNE.

Ces résultats montrent donc que cet actif diminue l'activité lipolytique des adipocytes.

### Exemple 2 : Mise en évidence des propriétés d'un autre hydrolysat de protéines de riz sur la lipolyse

Nous avons évalué l'effet d'un autre hydrolysat de protéines de riz (Colhibin® commercialisé par la société PENTAPHARM à 14.5% en matière active) sur l'hydrolyse des triglycérides adipocytaires en mesurant la libération d'acides gras non estérifiés (AGNE) dans le milieu.
Cet hydrolysat de protéines de riz comprend des peptides ayant une masse moléculaire moyenne allant de 1000 à 5000 daltons.

Nous avons suivi le même protocole que celui décrit à l'exemple 1 avec un milieu d'essai identique dans lequel ont été testés respectivement :
- l'hydrolysat de protéine de riz Colhibin® à la concentration finale de 10%, 2% et 0.4% correspondant respectivement à 1.45%, 0.29% et 0.058% de matière active ;
- la théophylline à la concentration finale de 1mM et l'isoprotérénol à la concentration finale de 1µM.

Nous avons obtenu les résultats suivants :

| **Traitement** | **Concentration finale dans le milieu** | **AGNE (µM)** | **% activité vs Témoin** | **p** |
|---|---|---|---|---|
| Témoin non traité | - | 33.2 | 100 | - |
| Hydrolysat de protéines de riz | 10% (1.45% de MA) | 4.0 | 12 | p<0.01 |
| | 2% (0.29% de MA) | 5.0 | 15 | p<0.01 |
| | 0.4% (0.058% de MA) | 13.0 | 39 | p>0.05 |
| Théophylline | 1 mM | 626.3 | 1888 | p>0.05 |
| Isoprotérénol | 1µM | 613.3 | 1849 | p>0.05 |

MA= matière active

Dans cet essai, la lipolyse basale correspondait à une libération de 33.2 µM d'acides gras non estérifiés (AGNE) en 2 heures dans la condition témoin non traité.
La Théophylline à 1mM et l'Isoprotérénol à 1 µM ont stimulé de façon significative la lipolyse (respectivement 1888 % et 1849 % du témoin), ce qui a permis de valider l'essai.
L'hydrolysat de protéines de riz Colhibin® a diminué significativement la lipolyse basale aux 3 concentrations testées, à 12%, 15% et 39% du témoin, et cela sans interférence apparente du produit avec le dosage des AGNE.

Ces résultats montrent donc que cet hydrolysat de protéines de riz diminue significativement l'activité lipolytique des adipocytes.

Les résultats des exemples 1 et 2 montrent donc que des hydrolysats de protéines de riz comprenant des peptides de faible masse moléculaire (inférieur à 1400 daltons) ou de poids moléculaire allant de 1000 à 5000 daltons diminuent significativement l'activité lypolitique des adipocytes.

### Exemple 3 : Sérum anti-âge

On prépare une émulsion H/E ayant la composition ci-dessous, de façon classique pour l'homme du métier :

| | |
|---|---|
| Hydrolysat de protéines de riz dans l'eau* | 0.5 % |
| Extrait de *Laminaria ochroleuca* dans un mélange eau/propylène glycol** | 0.1 % |
| Huiles | 5 % |
| Tensioactifs | 1 % |
| Agents tenseurs | 7 % |
| Epaississants / gélifiants | 1 % |
| Glycérine | 1 % |
| Propylène glycol | 1 % |
| Alcool | 13 % |
| Nacres | 0.7 % |
| Acide citrique | 0.1 % |
| Conservateurs | qs |
| Eau | qsp 100 % |

| | |
|---|---|
| * à 6.25% de matière active, préparé comme décrit dans la demande WO 02/102347 | |
| ** à 16.3% d'extrait sec (Laminaïne de SECMA) | |

Cette composition peut être appliquée matin et/ou soir sur le visage pour remodeler la peau et redessiner les contours du visage.

### Exemple 4 : Soin pour le contour des yeux

On prépare une émulsion H/E ayant la composition ci-dessous, de façon classique pour l'homme du métier :

| | |
|---|---|
| Hydrolysat de protéines de riz (Colhibin® Commercialisé par PENTAPHARM) | 0,075 % |
| C-β-D-xylopyranoside-2-hydroxy-propane | 2,5 % |
| Huiles | 8 % |
| Corps gras | 4 % |
| Tensioactifs | 2 % |
| Epaississants/ gélifiants | 1,5 % |
| Glycérine | 4 % |
| Neutralisants | 0,2 % |
| Conservateurs | 1,2 % |
| Charges | 3,5 % |
| Hexyldecanol | 1,5 % |
| Eau | qsp 100 % |

Cette composition peut être appliquée matin et/ou soir sur le contour des yeux pour limiter le creusement dû à l'âge.

## Revendications

1. Procédé cosmétique pour prévenir et/ou diminuer les signes du vieillissement cutané, par application sur la peau du visage d'une composition renfermant, dans un milieu physiologiquement acceptable, au moins un hydrolysat de protéines de riz et au moins un agent augmentant la synthèse des glycosaminoglycannes.

2. Procédé cosmétique selon la revendication 1, **caractérisé en ce qu'**il vise à remodeler le visage et/ou limiter le creusement du visage dû à l'âge.

3. Procédé cosmétique selon l'une des revendications 1 ou 2, **caractérisé en ce qu'**il vise à redessiner les contours du visage et/ou limiter le creusement des joues et/ou du contour de l'oeil.

4. Procédé cosmétique selon l'une quelconque des revendications précédentes, **caractérisé en ce que** les peptides contenus dans l'hydrolysat de protéines de riz ont une masse moléculaire inférieure ou égale à 5000 Daltons, de préférence inférieure ou égale à 3000 Daltons.

5. Procédé cosmétique selon l'une quelconque des revendications précédentes, **caractérisé en ce que** plus de 50% des peptides contenus dans l'hydrolysat de protéines de riz ont une masse moléculaire inférieure à 1400 Daltons.

6. Procédé cosmétique selon l'une quelconque des revendications précédentes, **caractérisé en ce qu'**au moins 40% des peptides ayant une masse moléculaire inférieure à 1400 Daltons ont une masse moléculaire comprise entre 75 et 175 Daltons.

7. Procédé cosmétique selon l'une quelconque des revendications précédentes, **caractérisé en ce que** les peptides contenus dans l'hydrolysat de protéines de riz ont une masse moléculaire moyenne comprise entre 300 et 400 Daltons.

8. Procédé cosmétique selon l'une quelconque des revendications précédentes, **caractérisé en ce que** ledit hydrolysat de protéines de riz est susceptible d'être obtenu selon un procédé de préparation comprenant les étapes consistant à :
- solubiliser des protéines de riz dans l'eau pour obtenir une suspension,
- hydrolyser cette suspension en présence d'une ou plusieurs enzymes choisies parmi : les aminopeptidases bactériennes en milieu neutre ou acide, les protéases stomacales d'origine animale en milieu acide, les protéases bactériennes en milieu neutre, les protéases bactériennes en milieu alcalin, extraites de *Bacillus licheniformis* ou de *Bacillus subtilis*, les endoprotéases bactériennes, et les enzymes pancréatiques d'origine animale ;
- avantageusement concentrer la fraction active en peptides de masse moléculaire inférieure à 5000 Daltons, de préférence en peptides de masse moléculaire inférieure à 1400 Daltons ;
- et éventuellement réaliser une filtration stérilisante de ce concentrat.

9. Procédé cosmétique selon l'une quelconque des revendications précédentes, **caractérisé en ce que** l'hydrolysat de protéines de riz provient de l'espèce *Oryza sativa.*

10. Procédé cosmétique selon la revendication 8, **caractérisé en ce que** le procédé de préparation dudit hydrolysat de protéines de riz ne comprend pas d'étape de fermentation ou de germination.

11. Procédé cosmétique selon l'une quelconque des revendications précédentes, **caractérisé en ce que** ledit hydrolysat de protéines de riz est issu d'une fraction de riz exempte de son de riz.

12. Procédé cosmétique selon l'une quelconque des revendications précédentes, **caractérisé en ce que** l'hydrolysat de protéines de riz représente de 0,01 à 2% en poids (en matière active), par rapport au poids total de la composition.

13. Procédé cosmétique selon l'une quelconque des revendications précédentes, **caractérisé en ce que** l'hydrolysat de protéines de riz représente de 0,02 à 0,4% en poids (en matière active), par rapport au poids total de la composition.

14. Procédé cosmétique selon l'une quelconque des revendications précédentes, **caractérisé en ce que** l'agent augmentant la synthèse des glycosaminoglycannes est choisi parmi : le D-xylose, ses esters et les oligosaccharides contenant du D-xylose ; les C-glycosides ; un produit de fermentation du lait par *Lactobacillus vulgaris* ; un extrait d'algue brune *Padina pavonica* ; un extrait de *Saccharomyces cerevisiae* ; un extrait de *Laminaria ochroleuca* ; un extrait de *Centella asiatica* ; et un extrait de cresson *(Nasturtium officinale*).

15. Procédé cosmétique selon l'une quelconque des revendications précédentes, **caractérisé en ce que** l'agent augmentant la synthèse des glycosaminoglycannes est un extrait de *Laminaria ochroleuca.*

16. Procédé cosmétique selon l'une quelconque des revendications 1 à 14, **caractérisé en ce que** l'agent augmentant la synthèse des glycosaminoglycannes est choisi parmi le C-β-D-xylopyranoside-2-hydroxypropane, ses sels et isomères optiques et géométriques.

17. Procédé cosmétique selon la revendication 15, **caractérisé en ce que** l'extrait de *Laminaria ochroleuca est* susceptible d'être obtenu suivant un procédé de préparation comprenant les étapes consistant à :
- récolter des algues fraîches,
- les laver,
- les broyer, avantageusement jusqu'à une taille comprise entre 100 et 200 µm,
- éliminer les débris cellulaires par centrifugation, pour obtenir un premier surnageant,
- fractionner le surnageant par précipitation à l'acide, et par adsorption sur charbon activé, pour obtenir un second surnageant,
- filtrer le second surnageant jusqu'à ce que le filtrat ait atteint une concentration en glycine-bétaïne, mesurée par HPLC, de 12.5% ± 2.5%.

18. Procédé cosmétique selon l'une quelconque des revendications précédentes, **caractérisé en ce que** l'agent augmentant la synthèse des glycosaminoglycannes représente de 0,001 à 5% en poids (en matière active), par rapport au poids total de la composition.

19. Procédé cosmétique selon l'une quelconque des revendications 15 à 17, **caractérisé en ce que** l'extrait de *Laminaria ochroleuca* représente de 0,005 à 0,1 % (en poids d'extrait sec) du poids total de la composition.

20. Procédé cosmétique selon l'une quelconque des revendications précédentes, **caractérisé en ce que** l'hydrolysat de protéines de riz et l'agent augmentant la synthèse des glycosaminoglycannes sont incorporés ensemble, ou chacun séparément, dans des niosomes.

21. Procédé cosmétique selon l'une quelconque des revendications précédentes, **caractérisé en ce que** la composition se présente sous la forme d'une émulsion huile-dans-eau.

22. Composition cosmétique susceptible d'être utilisée dans le procédé selon l'une quelconque des revendications précédentes renfermant, dans un milieu physiologiquement acceptable, au moins un hydrolysat de protéines de riz et au moins un extrait de *Laminaria ochroleuca.*

23. Composition cosmétique susceptible d'être utilisée dans le procédé selon l'une quelconque des revendications précédentes renfermant, dans un milieu physiologiquement acceptable, au moins un hydrolysat de protéines de riz et au moins un agent choisi parmi le C-β-D-xylopyranoside-2-hydroxy-propane, ses sels et isomères optiques et géométriques.

24. Utilisation cosmétique d'au moins un hydrolysat de protéines de riz associé à au moins un agent augmentant la synthèse des glycosaminoglycannes dans une composition, comme agent destiné à remodeler le visage et/ou limiter le creusement du visage dû à l'âge.
